(19) Europäisches Patentamt
European Patent Office
Office européen des brevets

(11) Publication number: 0 049 610
A1

# (12) EUROPEAN PATENT APPLICATION

(21) Application number: 81304557.2

(22) Date of filing: 01.10.81

(51) Int. Cl.[3]: C 07 D 487/04
//(C07D487/04, 209/00, 205/00)

(30) Priority: 07.10.80 JP 140125/80

(43) Date of publication of application:
14.04.82 Bulletin 82/15

(84) Designated Contracting States:
AT DE FR GB IT NL SE

(71) Applicant: KOWA COMPANY, LTD.
6-29, 3-chome Nishiki
Naka-ku Nagoya-shi Aichi-ken(JP)

(72) Inventor: Nakayama, Masahito
Green Town 2-801 3-1293-10 Tateno
Higashi-Yamato-shi Tokyo(JP)

(72) Inventor: Kimura, Shigeru
4-51-1, Kashiwa-cho
Tachikawa-shi Tokyo(JP)

(72) Inventor: Mizoguchi, Toshimi
2-17-43, Noguchi-cho
Higashi-Murayama-shi Tokyo(JP)

(72) Inventor: Tanabe, Sohei
2-17-43, Noguchi-cho
Higashi-Murayama-shi Tokyo(JP)

(72) Inventor: Mori, Toshihito
4-16-3, Fujimi-cho
Higashi-Murayama-shi Tokyo(JP)

(74) Representative: Webb, Frederick Ronald et al,
G.F. Redfern & Co. Marlborough Lodge 14 Farncombe Road
Worthing West Sussex BN11 2BT(GB)

## (54) A novel carbapenem compound, its esters and salts, and processes for producing them.

(57) A carbapenem compound of the formula,

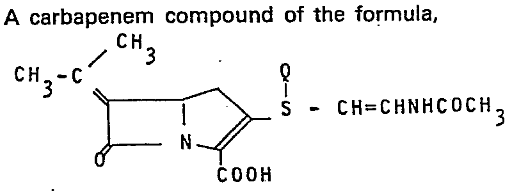

and esters and salts thereof are effectively useful as intermediates for the preparation of medicinal substances exhibiting antibacterial and β-lactamase-inhibitory activities. These compounds may be produced by esterifying a substance of the formula:

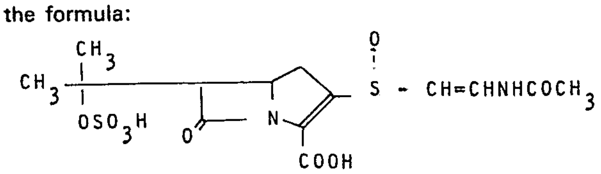

and subjecting the resulting ester to a reaction for the elimination of sulphuric acid and, if necessary, thereafter then to a de-esterification treatment.

EP 0 049 610 A1

Croydon Printing Company Ltd

"A NOVEL CARBAPENEM COMPOUND, ITS ESTERS AND SALTS, AND PROCESSES FOR PRODUCING THEM"

This invention relates to a novel carbapenem compound and its esters and salts, and more particularly, to a carbapenem compound represented by the following formula (I)

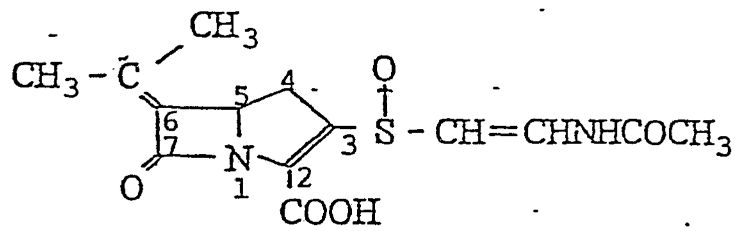

(I)

and its esters and salts, and also to processes for producing the same.

The present Applicants have succeeded in isolating the substance KA-6643-B showing antibacterial activities and β-lactamase-inhibitory activity as a fermentation metabolite, and which is represented by the formula (II) below, from a culture broth of a strain of Streptomyces sp. KC-6643 belonging to the genus Streptomyces. This new strain was deposited with the Fermentation Research Institute of the Agency of Industrial Science & Technology located at No. 1-3, Higashi 1-chome, Yatabe-machi, Tsukuba-gun, Ibaragi-ken, Japan, on April 7, 1978, and has been given Acceptance No. FERM-P 4467. The strain was also deposited with the American Type Culture Collection located at No. 12301, Parklawn Drive, Rockville, Maryland, U.S.A., on March 12, 1979, and has been given ATCC Acceptance No. ATCC 31493. The isolation of the substance KA-6643-B is described in Japanese Patent Application No. 54-119139, as laid open under No. 56-43284, and in published U.K. Patent Application No. 2,047,700A, both in the name of the present Applicants.

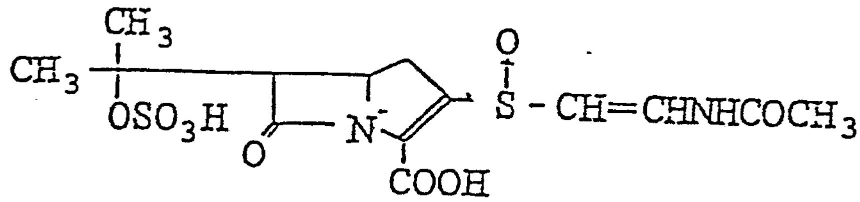

(II)

The present Applicants have made a further investigation of this substance KA-6643-B and, as a result, have found that the novel compound of the formula (I) can be obtained through the elimination of sulphuric acid from the substance and that this compound (I) is useful as an intermediate for preparing medicines showing antibacterial and β-lactamase-inhibitory actions. The present invention is based on this finding.

It is accordingly, an object of the invention to provide a novel carbapenem compound, and esters and salts thereof which are effectively useful as intermediates for the preparation of medicines exhibiting antibacterial and β-lactamase-inhibitory activities.

Another object of the invention is to provide a process for producing such a carbapenem compound, and its esters and salts.

According to the invention, there is provided the carbapenem compound represented by the following formula (I)

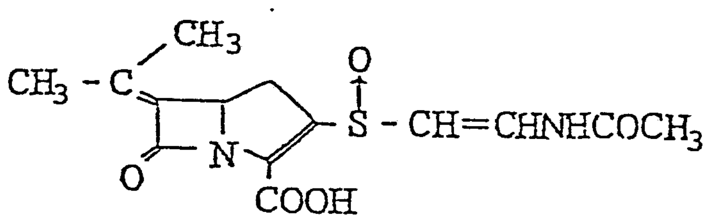

(I)

at the carboxyl group thereof.

The carbapenem compound (I), and its esters and salts according to the invention can be prepared by esterifying the substance KA-6643-B of the formula (II) at the carboxyl group thereof, and subjecting the

resulting ester to a reaction for the elimination of sulphuric acid and, if the compound itself is required, then to an optional de-esterification treatment.

The esterification of the carboxyl group in the process of the invention can be effected by any of ordinary methods including, for example, a method in which alcohols, or their reactive derivatives, are reacted with KA-6643-B, or its reactive derivatives, at the carboxyl group thereof. Preferred esters include the methyl ester, ethyl ester, phenyl ester, benzyl ester, p-nitrobenzyl ester, acetoxymethyl ester and the like.

The reaction for the elimination of sulphuric acid may be carried out in an aqueous solution under from weakly acidic to strongly basic conditions at a temperature of from 30 - 150°C for 0.5 - 10 hours.

The acids which can be used in this reaction include mineral acids, such as phosphoric acid, hydrochloric acid, sulphuric acid and the like, and organic acids, such as acetic acid, citric acid, oxalic acid and the like. Representative alkalis are sodium hydroxide, barium hydroxide, ammonia and the like.

Alternatively, the elimination of sulphuric acid may be effected by contacting the ester of the compound of formula (II) with an ion exchange resin. Examples of suitable ion exchange resins include strongly acidic cation exchange resins, such as Amberlite IRA-410 and IR-120 (Rohm & Haas Co., Ltd.), Dowex 50 (Dow Chem., Co., Ltd.), and the like, and weakly acidic cation exchange resins such as Amberlite IRC-50, IRC-84 and CG-50, and Diaion WK-10 and WK-20 (Mitsubishi Chem. Ind. Ltd.).

The de-esterification when required is conducted using known techniques such as hydrolysis, or hydrogenation.

The isolation and purification of the compound of

the formula (I) from the reaction solution can be effected by any ordinary methods. Preferably, there are used gel filtration, column chromatography, reversed phase chromatography, freeze drying, or the like, which methods may be used singly or in combination.

The free carbapenem compound (I) so obtained may if desired, be converted into an alkali metal salt, an alkaline earth metal salt, a primary, secondary or tertiary amine salt, or a quaternary ammonium salt by conventional methods.

The compound (I) of the invention which has been obtained as previously described can be converted into compounds showing antibacterial and β-lactamase-inhibitory actions. For example, a compound represented by the formula (III) is obtained when the compound of the formula (I) is reduced :

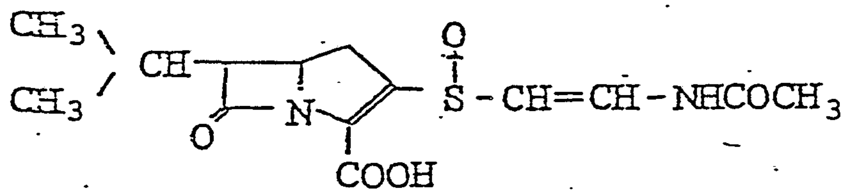

(III)

and a compound of the formula (IV) is obtained when the compound of the formula (I) is reacted with ethanethiol:

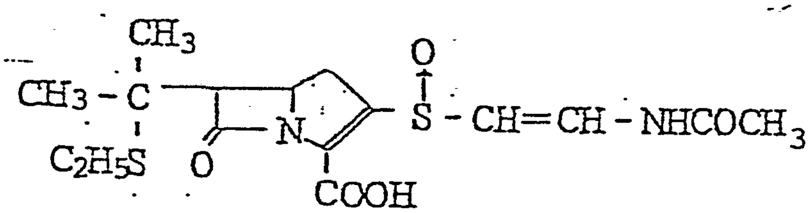

(IV)

The invention is illustrated by the following non-limitative examples :-

Example 1

20 mg of the disodium salt of KA-6643-B was reacted with 12 mg of p-nitrobenzyl bromide in 0.75 mℓ of dimethylformamide at room temperature for 2 hours. After completion of the reaction, the solvent was distilled off under reduced pressure and the resulting residue was purified by preparative thin layer chromatography (carrier: silica $HF_{254}$; solvent: methylene chloride-methanol (4:1) to obtain 12.3 mg of the paranitrobenzyl ester of KA-6643-B.

UV values: $\lambda_{max}^{MeOH}$ nm

240, 305 (shoulder)

IR values: $\gamma_{max}^{KBr}$ $cm^{-1}$

1780, 1705,1680, 1615

NMR values: $\delta$ $DMF-d_7$ ppm

1.58 (3H, s) 1.60 (3H, s)
2.06 (3H, s) 3.99 (1H, d, J=6Hz)
4.36 (1H, m) 5.32 (1H, d, J=15Hz)
5.50 (1H, d, J=15Hz)
6.28 (1H, d, J=15Hz)
7.38 (1H, dd, J=12, 15Hz)
7.72 (2H, d, J=9Hz)
8.16 (2H, d, J=9Hz)
10.44 (1H, brd, J=12Hz)

Example 2

3.7 mg of the ester obtained in Example 1 was dissolved in 0.25 mℓ of a 0.1M phosphate buffer solution having a pH of 7.0 and allowed to stand at 60°C for 2 hours.

After cooling the reaction solution, it was extracted with ethyl acetate, washed with a saturated

saline solution, and the solvent was distilled off under reduced pressure to obtain 2.25 mg of the p-nitrobenzyl ester of (5R, 6R)-3-[(E)-2-acetamidoethenylsulfinyl]-6-isopropylidene-7-oxo-1-azabicyclo[3.2.0]hepto-2-en-2-carboxylic acid.

UV values: $\lambda_{max}^{MeOH}$ nm
247, 285 (shoulder), 327 (shoulder)

IR values: $\gamma_{max}^{KBr}$ $cm^{-1}$
1780, 1720, 1700, 1670, 1640, 1605

NMR values: δ $DMSO-d_6$ ppm
1.85 (3H, s) 2.00 (3H, s)
2.06 (3H, s) 3.20 (2H, m)
4.92 (1H, t, J=9Hz) 5.28 (2H, s)
6.24 (1H, d, J=14Hz)
7.36 (1H, dd, J=10, 14Hz)
7.78 (2H, d, J=9Hz)
8.30 (2H, d, J=9Hz)

Example 3

20 mg of the p-nitrobenzyl ester of (5R, 6R)-3-[(E)-2-acetamidoethenylsulfinyl]-6-isopropylidene-7-oxo-1-azabicyclo [3.2.0] hepto-2-en-2-carboxylic acid was dissolved in a mixture of 1.5 mℓ dioxane and 0.5 mℓ 2-propanol, to which were added 2 mℓ of 0.1M phosphate buffer solution (pH: 7.0) and 10% palladium-charcoal so as to effect a hydrogenation reaction under a pressure of 3.5 $kg/cm^2$ for 60 minutes.

The reaction mixture was filtered and the catalyst was washed with the above-indicated buffer solution. The resulting filtrate and washings were washed with ethyl acetate and then concentrated to about 2 mℓ . The concentrate was passed through a column (1 x 5 cm)

of Diaion HP-20 for adsorption, and then eluted with deionized water. The eluate was subjected to bioassay using *Comamonas terrigena* as mycelia to be tested, thereby collecting an active fraction and then concentrating it. The resulting concentrate was freeze-dried to obtain 4 mg of sodium (5R, 6R)-3-[(E)-2-acetamidoethenylsulfinyl]-6-isopropylidene-7-oxo-1-azabicyclo[3.2.0]hepto-2-en-2-carboxylate.

UV values: $\lambda_{max}^{H_2O}$ nm

248,350

CLAIMS :-

1. A carbapenem compound represented by the following formula (I), and esters and salts thereof:

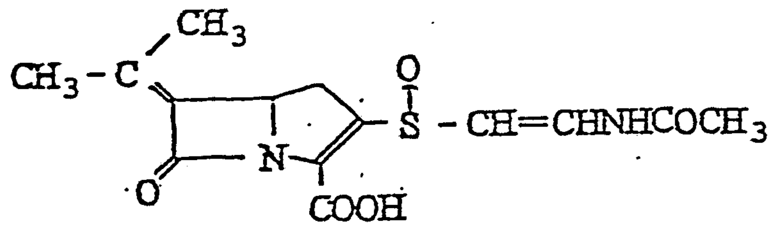

(I)

2. A process for producing the carbapenem compound of Claim 1, or an ester or salt thereof characterized by the steps of esterifying a KA-6643-B substance represented by the following formula (II):

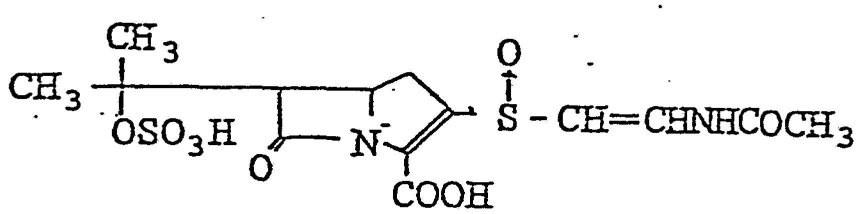

(II)

at the carboxyl group thereof, and subjecting the resulting ester to a reaction for the elimination of sulphuric acid and optionally thereafter to de-esterification.

0049610

European Patent Office

# EUROPEAN SEARCH REPORT

Application number
EP 81 30 4557

| Category | DOCUMENTS CONSIDERED TO BE RELEVANT<br>Citation of document with indication, where appropriate, of relevant passages | Relevant to claim |
| --- | --- | --- |
| | EP - A - 0 005 349 (BEECHAM)<br>* claims 1,12,13 and 23 *<br>--- | 1,2 |
| A | DE - A - 2 815 157 (MERCK)<br>* claims 2,6 and 7 *<br>--- | 1,2 |
| P | EP - A - 0 017 246 (SHIONOGI)<br>* page 2, 2nd paragraph *<br>---------- | 1 |

CLASSIFICATION OF THE APPLICATION (Int Cl.³)
C 07 D 487/04//
(C 07 D 487/04
209/00
205/00)

TECHNICAL FIELDS SEARCHED (Int. Cl.³)
C 07 D 487/04

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
| --- | --- | --- |
| The Hague | 15.12.1981 | CHOULY |

EPO Form 1503.1 06.78